**Europäisches Patentamt**

**European Patent Office**

**Office européen des brevets**

(11) Publication number: **0 251 710**
**A2**

## (12) EUROPEAN PATENT APPLICATION

(21) Application number: **87305671.7**

(22) Date of filing: **25.06.87**

(51) Int. Cl.⁴: **A61K 39/395** , **C07K 15/08** , **C12P 21/00**

(30) Priority: **26.06.86 JP 148072/86**
**22.07.86 JP 170788/86**

(43) Date of publication of application:
**07.01.88 Bulletin 88/01**

(84) Designated Contracting States:
**AT BE DE FR GB NL**

(71) Applicant: **BIO-SCIENCE LABORATORY**
**34-5 Shironishimachi 5-chome**
**Yamagata-shi Yamagata 990(JP)**

(72) Inventor: **Oikawa, Taneaki**
**c/o Bio Science Lab. 34-5 Shironishimachi**
**5-chome**
**Yamagata-shi Yamagata 990(JP)**

(74) Representative: **Armitage, Ian Michael et al**
**MEWBURN ELLIS & CO. 2/3 Cursitor Street**
**London EC4A 1BQ(GB)**

(54) **Antibody against male-specific antigen and process for production thereof.**

(57) An antibody against a male-specific antigen is produced. which antibody specifically bonds to the middle piece of Y chromosome bearing spermatozoon. The production process includes removing from a male animal a part of its body, such as cells, tissue or organ, the body part being one in which a male-specific antigen is produced. The removed part of the body is then homogenized. and the homogenate optionally mixed with an adjuvant to form an antigenic preparation. A female animal is immunized with the antigenic preparation, blood obtained from the immunized female animal, and the target antibody recovered from the blood. The antibody may be utilized in a process for discrimination between X chromosome bearing spermatozoa and Y chromosome bearing spermatozoa by placing the obtained antibody in contact with a sperm sample and detecting spermatozoa which bond with the antibody at the middle piece.

*Fig. 5*

(B)

QUINACRINE FLUORESCENCE

(A)

(C)

ANTIBODY BONDING SIDE

EP 0 251 710 A2

## ANTIBODY AGAINST MALE-SPECIFIC ANTIGEN AND PROCESS FOR PRODUCTION THEREOF

The present invention relates to a male-specific antigen, a process for the production thereof, and the use thereof.

In skin graft experiments, it has been found that although a homosexual graft and a graft from a female animal to a male animal are not rejected, a graft from a male animal to a female animal is rejected (Eichwald, E.J. and Silmser, C.R., Transp. Bull. 2, l48, l955). Since this phenomenon is caused by an expression product of a gene present on the Y chromosome, this product is designated as a "histocompatibility Y chromosome linked antigen; or H-Y antigen", or as a "male-specific antigen" including the H-Y antigen.

It is known that an antibody against the male-specific antigen can be prepared by immunizing a female mouse with the spleen or testis of a male mouse, which mice are inbred, and recovering the antibody from the immunized female mouse (Wachtel, S.S., Koo, G.C. Zuckerman, E.E., Hammerling, U., Scheid, M.M., and Boyse, E.A., Proc. Natl. Acad. Sci. U.S.A., Vol 7l, l2l5, l974; and Utsumi, K., Sato, E., and Yuhara, M., J. Reprod. Immunol., Vol 5, Sup. l, 59, l983). However, the specificity of antibodies prepared by such processes has not been studied in detail, and in particular it has not been determined whether these antibodies bind to male spermatozoa. Inbred mice, and more generally, inbred animals are bred and maintained by mass mating, and therefore, do not have a high coefficient of relationship in a gene combination. Therefore, it is assumed that the specificity of an antibody prepared using such inbred animals is not high.

Proc. Natl. Acad. Sci. U.S.A., Vol 70, No. 5, l502 - l505, l973 discloses that an antiserum prepared from a female mouse which has rejected skin grafting from a male mouse binds to the head of spermatozoon. However, this antiserum is different from the present antibody in that the present antibody specifically binds to the middle piece of spermatozoon.

The present invention provides an antibody against male-specific antigen characterized in that the antibody specifically binds to the middle piece of male sperm.

The present invention also provides a process for a production of an antibody comprising the steps of:

removing from a male animal a part of its body including cells, tissue, and/or organ, a male-specific antigen being produced in that part of the body;

homogenizing the removed part of the body and, optionally, mixing the homogenate with an adjuvant to form an antigenic preparation;

immunizing a female animal with the antigenic preparation;

obtaining blood from the immunized female animal; and

recovering the target antibody from the blood.

Moreover, the present invention provides a process for discrimination between Y chromosome bearing spermatozoa and X chromosome bearing spermatozoa comprising

placing the obtained antibody in contact with a sperm sample; and

detecting spermatozoa which bond with the antibody at the middle piece of the spermatozoa.

Figure lA represents a visible light micrograph of spermatozoa of a hamster, which spermatozoa have been treated with the present antibody and then with rabbit anti-hamster IgG labelled with fluorescein isothiocyanate (FITC), according to Example 2;

Fig. lB represents a fluorescence micrograph of the hamster spermatozoa corresponding to Fig. lA;

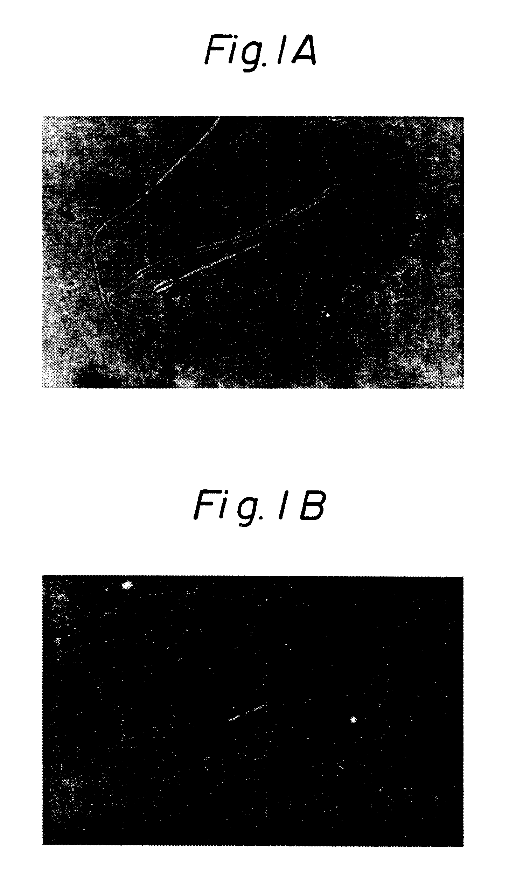

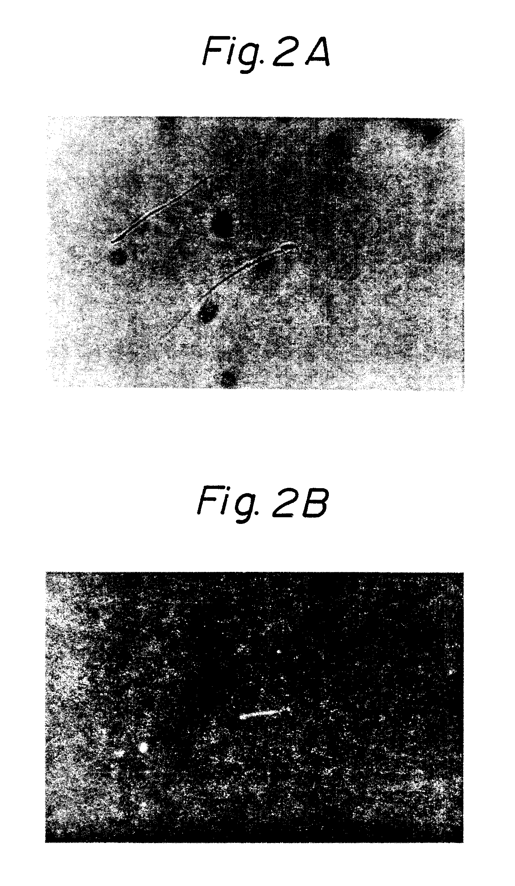

Fig. 2A represents a visible light micrograph of spermatozoa of a mouse prepared by the same procedure as in Fig. lA;

Fig. 2B represents a fluorescence micrograph of the mouse spermatozoa corresponding to Fig. 2A;

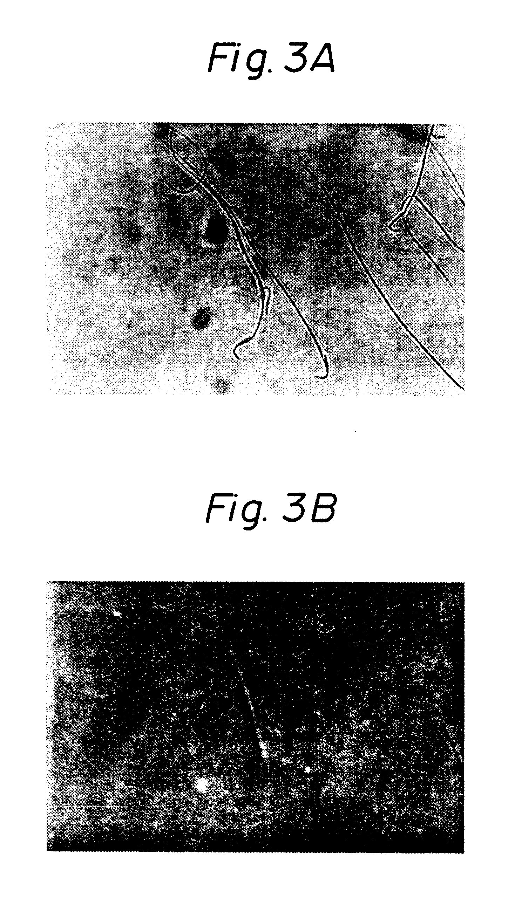

Fig 3A represents a visible light micrograph of spermatozoa of a rat prepared by the same procedure as in Fig. lA;

Fig. 3B represents a fluorescence micrograph of the rat spermatozoa corresponding to Fig. 3A;

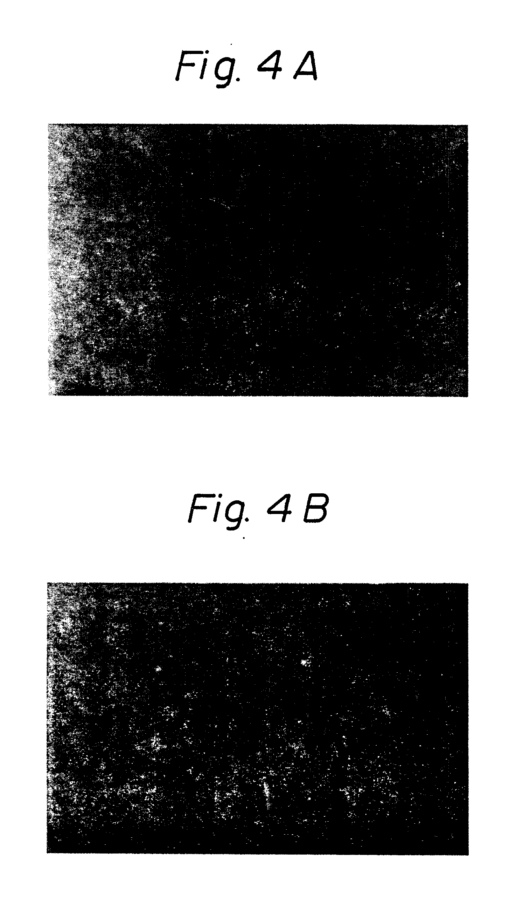

Fig 4A represents a visible light micrograph of spermatozoa of bovine prepared by the same procedure as in Fig. lA;

Fig. 4B represents a fluorescence micrograph of the bovine spermatozoa corresponding to Fig. 4A; and,

Fig. 5 represents a sketch of spermatozoa of human which have been treated with the present antibody, biotinylated anti-hamster IgG, rhodamine-labelled avidin, and then quinacrine mustard according to Example 3.

The present antibody against a male-specific antigen is prepared by using isogenic animals. Any kind of animal, such as a mouse, rat, hamster, rabbit, pig, guinea pig, or bovine can be used, provided that the animal is isogenic. Isogenic animals have a more than 99.8% coefficient of relationship, and male animals and female animals have a same gene combination for all genes other than a gene for the male-specific antigen. The isogenic animals are obtained by sib mating through about 20 generations. In the sib mating, a pair of a male animal and a female animal born from same parents is separated from other animals and bred. Therefore, the sib mating is completely different from mass-mating wherein more than one pair of animals are bred.

As described above, since in the isogenic animals a male animal and a female animal have a same gene combination, except that the male animal has a gene for male-specific antigen, if the female animal is immunized by an organ or tissue of the male animal in which the gene for the male-specific antigen is expressed, the female animal produces only an antibody against the male-specific antigen.

Therefore, according to the present invention, a female animal is immunized with an immunogenic preparation containing male-specific antigen. This preparation may be prepared from cells, tissue or organ wherein the male specific antigen is expressed, such as testicle, spleen, brain and the like. The testicle is especially preferable. Preferably, the cells, tissue or organ have been homogenated. The homogenate is then mixed with an adjuvant such as the complete Freund's adjuvant in the same volume ratio, and the mixture is injected to a female animal. The immunization is usually carried out by subcutaneous, intracutaneous or peritoneal injection, or direct injection into the spleen. The immunization is carried out about five times, at weekly intervals.

About ten days after the final immunization, blood is obtained from the immunized animal, and the desired antibody is recovered from the blood.

The present antibody against the male-specific antigen can bond to a middle piece of the spermatozoon of various kinds of mammals, such as a hamster, mouse, rat, pig, human and the like. To confirm such bonding, for example, spermatozoa to be tested are washed by centrifugation, and incubated with the antibody of the present invention in a buffer such as an isotonic buffer or Tyrode's solution containing 5% bovine serum albumin, and again washed with the buffer to remove free-antibody or nonspecifically bonded antibody from the spermatozoa. Next, the spermatozoa are incubated with rabbit anti-hamster IgG labelled with fluorescein isothiocyanate (FITC), and again washed. The spermatozoa thus prepared are observed under a fluorescence microscope. In this observation, the present antibody against the male-specific antigen bond to about 50% of spermatozoa of not only a hamster but also a mouse, rat, pig, bovine, and human.

It is known that, among human spermatozoa, those which have the Y chromosome, i.e., Y chromosome bearing spermatozoa, are stained with quinacrine mustard to reveal a brilliant point designated as an F-body (Barlow et al. Nature, London, 226, 261-262, 1970). When human spermatozoa are double stained by immuno fluorescence stain using the present antibody and by quinacrine mustard stain, only those spermatozoa which shown an F-body are stained by the immuno fluorescence stain.

This means that the present antibody against male-specific antigen specifically bonds to Y chromosome bearing spermatozoon of various kinds of animals including not only a hamster but also a mouse, rat, pig, and human, and that the present antibody is of the IgG class.


## Example

The present invention will now be further illustrated by, but is no means limited to, the following examples.


## Example I Production of antibody against male-specific antigen

Testicles of isogenic male hamsters prepared by sib-mating over 20 generations were removed, and homogenized by a commercially available glass homogenizer, and the homogenate was mixed with an equal volume of complete Freund's adjuvant to form an immunogenic emulsion. I ml of the emulsion was subcutaneously injected to two isogenic female hamsters to immunize them. The same injections were carried out five times at weekly intervals. After 10 days from the final injection, the hamsters were sacrificed by cervical vertebral dislocation, and about 8 ml of blood was collected from the hearts of the animals.

The blood was allowed to stand at 4°C over night for coagulation, and centrifuged at 0°C and 20,000 $\times$g for 10 minutes to obtain 4 ml of antiserum. The antiserum thus obtained was heated at 56°C for 30 minutes to obtain an antiserum containing the antibody against the male-specific antigen.

Example 2  Sexual differentiation of spermatozoa from various animals

Sperm samples from hamster, mouse, rat, bovine, pig, and human were separately washed 2 to 3 times with a isotonic phosphate buffer (pH 7.4) or Tyrode's solution containing 5% bovine serum albumin by centrifugation, each for l0 minutes at 250 to 300 $^\times$g, to prepare a spermatozoa suspension in Tyrode's solution or isotonic phosphate buffer. The suspension was then mixed with the serum containing the present antibody, and the mixture was incubated at 37°C for 30 minutes. Next, the spermatozoa were washed one to two times with Tyrode's solution or isotonic phosphate buffer by centrifugation, each for l0 minutes, at 250 to 300 $^\times$g to eliminate a free-antibody and non-specifically bonded antibody from the spermatozoa. Next, the spermatozoa were treated with rabbit anti-hamster IgG labelled with FITC at 37°C for 30 minutes. The treated spermatozoa were then washed with an isotonic phosphate buffer or Tyrode's solution containing 5% bovine serum albumin by centrifugation at 250 to 300 $^\times$g for l0 minutes. The thus-prepared spermatozoa were mounted on a non-fluorescent slide glass with four spots of vaselne, and the slide glass was covered by a cover glass, and sealed with manicure to obtain a specimen for fluorescence microscopy. The thus-obtained specimen was observed under a fluorescence microscope, and it was found that about half the number of spermatozoa bonded to the present antibody.

Moreover, since the present antibody was detected by rabbit anti-hamster IgG, the antibody prepared as described above was proved to be of the IgG class.

The results are shown in Figs. l to 4. Figures l to 4 are visible light micrographs (A) and fluorescence micrographs (B) obtained by observing a same visual field of the specimen obtained as described above. From these micrographs, it is seen that, among spermatozoa present in a visual field, a proportion of spermatozoa show fluorescence at their middle piece, revealing that the present antibody specifically bonds to the middle piece.

Example 3

Spermatozoa of a human were washed according to the procedure as described in Example 2, and the washed spermatozoa were fixed in a solution containing 2% paraformaldehyde, 75 mM lysine, l0 mM sodium metaperiodate and 32.5 mM phosphate buffer (pH 6.2) at 4°C for an hour, and the fixed spermatozoa were washed at least two times with an isotonic phosphate buffer by centrification. A slide glass was coated with the spermatozoa preparation and dried. Next, the spermatozoa adhered on the slide glass were treated as follows.

The spermatozoa were first incubated in an isotonic phosphate buffer containing 5% bovine serum albumin at 37°C for an hour, and then in a serum containing the present antibody at 37°C for 30 minutes, in a biotinated anti-hamster IgG preparation at 37°C for 30 minutes, in rhodamine-labelled avidin at 37°C for 30 minutes, and finally, in 0.005% quinacrine mustard at a room temperature for 30 minutes. During these incubations, before each incubation the slide glass was washed with an isotonic phosphate buffer. After the final washing, the slide glass was covered with a cover glass.

The results are shown in Table I. Three experiments were carried out using three sperm samples obtained from three men, and the results obtained were tested by $\chi^2$ square As a result, it was found that there was no significant difference between the three samples for each experiment. Moreover, the results of three different experiments were summed up for $\chi^2$ square. As a result, spermatozoa which were immunologically stained were also stained with quinacrine mustard with a more than 0.l% accuracy. Therefore, it is concluded that the present antibody against the male-specific antigen recognizes only Y chromosome bearing spermatozoa.

## Table 1

### Double staining of human spermatozoa

| | | | | |
|---|---|---|---|---|
| Immuno fluorescence stain | + | + | − | − |
| Quinacrine stain | + | − | + | − |
| Experiment 1 | 216 (43.81) | 16 (3.25) | 51 (10.34) | 210 (42.60) |
| Experiment 2 | 153 (38.93) | 28 (7.12) | 38 (9.67) | 174 (44.27) |
| Experiment 3 | 162 (38.76) | 30 (7.18) | 35 (8.37) | 191 (45.69) |
| Total | 531 (40.72) | 74 (5.67) | 124 (9.51) | 575 (44.10) |

In Table I, the case wherein spermatozoon was stained is shown by the symbol +, and the case wherein spermatozoon was stained is shown by the symbol -. The numbers in parenthesis shown percentages of the number of spermatozoa corresponding to the combination of two symbols on the basis of the total number of spermatozoa observed in the experiment. Each experiment was sepa rately carried out using sperm samples obtained from different individuals.

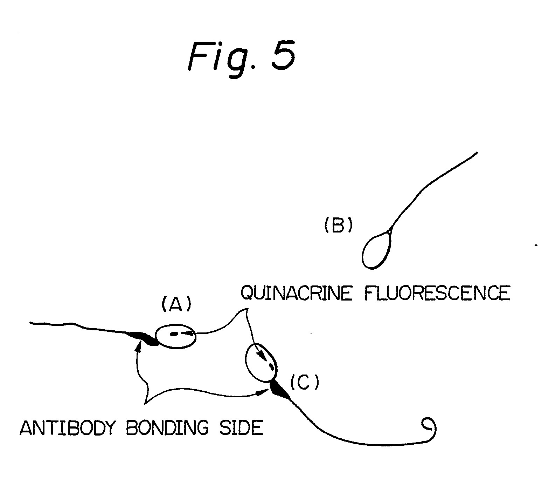

A sketch of a fluorescence micrograph is shown in Fig. 5, wherein spermatozoa (A) and (C), which are thought to be male spermatozoa due to a positive result of the quinacrine staining, were also stained at their middle piece by immuno fluorescence stain. On the other hand, spermatozoon (B), which was thought to be female spermatozoon due to a negative result of the quinacrine staining was not stained by the immuno fluorescence stain.

As seen from the above-mentioned result, the present antibody against the male-specific antigen is easily produced, and can be used to discriminate between Y chromosome bearing spermatozoa and X chromosome bearing spermatozoa, and moreover, can be used to fractionate Y chromosome bearing spermatozoa and X chromosome bearing spermatozoa.

**Claims**

1. An antibody against a male-specific antigen characterized in that the antibody specifically bonds to the middle piece of Y chromosome bearing spermatozoon.

2. A process for production of the antibody of claim 1 comprising the steps of:

removing from a male animal a part of its body including cells, tissue and/or organ, a male-specific antigen being produced in that part of the body;

homogenizing the removed part of the body and optionally mixing the homogenate with an adjuvant to form an antigenic preparation;

immunizing a female animal with the antigenic preparation;

obtaining blood from the immunized female animal; and

recovering the target antibody from the blood.

3. A process according to claim 2, wherein the animal is a hamster.

4. A process according to claim 2 or claim 3. wherein the animal is isogenic.

5. A process for discrimination between Y chromosome bearing spermatozoa and X chromosome bearing spermatozoa comprising

placing the antibody of claim 1 in contact with a sperm sample; and

detecting spermatozoa which bond with the antibody at their middle piece.

Claims for the following Contracting State: AT

1. A process comprising production of an antibody against a male-specific antigen characterized in that the antibody specifically bonds to the middle piece of Y chromosome bearing spermatozoon.

2. A process according to claim 1 comprising the steps of:

removing from a male animal a part of its body including cells, tissue and/or organ, a male-specific antigen being produced in that part of the body;

homogenizing the removed part of the body and, optionally mixing the homogenate with an adjuvant to form an antigenic preparation;

immunizing a female animal with the antigenic preparation;

obtaining blood from the immunized female animal; and

recovering the target antibody from the blood.

3. A process according to claim 2, wherein the animal is a hamster.

4. A process according to claim 2 or claim 3, wherein the animal is isogenic.

5. A process for discrimination between Y chromosome bearing spermatozoa and X chromosome bearing spermatozoa comprising

placing the antibody of claim 1 in contact with a sperm sample; and

detecting spermatozoa which bond with the antibody at their middle piece.

## Fig. IA

## Fig. I B

## Fig. 2 A

## Fig. 2B

*Fig. 3A*

*Fig. 3B*

*Fig. 4 A*

*Fig. 4 B*

# Fig. 5

(B)

QUINACRINE FLUORESCENCE

(A)

(C)

ANTIBODY BONDING SIDE